# EUROPEAN PATENT APPLICATION

(11) **EP 3 871 503 A1**
(43) Date of publication of application: **01.09.2021**
(21) Application number: 21158141.8
(22) Date of filing: 19.02.2021
(51) Int. Cl.: A01N 59/12

(54) **SYSTEMS AND METHODS FOR STERILIZING IODOPHOR COMPOSITIONS**

(30) Priority: 26.02.2020 US 202062981964 P
(71) Applicant: CareFusion Corporation, San Diego, CA 92130 (US)
(72) Inventor: Sieracki, Nathan, Chicago, IL 60613 (US); McGinley, Christopher, Highland Park, IL 60035 (US); Ortiz, Moises, Gurnee, IL 60031 (US)
(74) Representative: Hoffmann Eitle

(57) **Abstract**

Systems and methods for sterilizing iodophor compositions are described. In some cases, the iodophor composition comprises a complex comprising iodine and a polymer (i.e., an iodine-polymer complex). The iodine-polymer complex may be polyvinylpyrrolidone iodine (also referred to as "povidone-iodine" or "PVP-I") or iodine povacrylex. Methods are described for heating an initial iodophor composition to produce a sterilized iodophor composition. The resulting sterilized iodophor composition may have a relatively high assurance of sterility (e.g., a sterility assurance level of 10⁻³ or less, 10⁻⁶ or less). In some cases, heating the initial iodophor composition comprises exposing at least a portion of a container containing the initial iodophor composition to a heated liquid. In certain cases, the heated liquid is part of a moving liquid stream (e.g., a cascading waterfall stream produced by a cascading waterfall sterilizer). According to some embodiments, it was surprisingly determined that heating an initial iodophor composition even to a relatively low maximum temperature (i.e., a "sterilization temperature") while maintaining the sterilization temperature for a relatively short amount of time (i.e., a "sterilization time") can still lead to a relatively high assurance of sterility using certain methods described herein. As a result, sterilization systems and methods described herein may advantageously preserve certain positive attributes of an initial iodophor composition (e.g., potency, pH, viscosity).

## Description

### TECHNICAL FIELD

Systems and methods for sterilizing iodophor compositions are generally provided.

### BACKGROUND

Sterilization of antiseptic compositions, including iodophor compositions, can advantageously reduce and/or prevent infection. However, known methods of sterilizing antiseptic compositions, such as exposure to gamma radiation, can have deleterious effects on iodophor compositions. Accordingly, improved systems and methods for sterilizing iodophor compositions are needed.

### SUMMARY

Systems and methods for sterilizing iodophor compositions are generally provided. The subject matter disclosed herein involves, in some cases, interrelated products, alternative solutions to a particular problem, and/or a plurality of different uses of one or more systems and/or articles.

In some aspects, a sterilized iodophor composition is provided. In some embodiments, the sterilized iodophor composition is sterilized by a method comprising heating an initial iodophor composition to produce the sterilized iodophor composition. In certain embodiments, the sterilized iodophor composition has a sterility assurance level of 10⁻³ or less.

In some aspects, a sterilized iodophor composition is provided. In some embodiments, the sterilized iodophor composition comprises at least 5.0% by weight of povidone-iodine. In certain embodiments, the sterilized iodophor composition has a sterility assurance level of 10⁻³ or less. In certain embodiments, the sterilized iodophor composition has a viscosity of 3000 cP or less.

In some aspects, a sterilization method is provided. In some embodiments, the sterilization method comprises heating an initial iodophor composition to produce a sterilized iodophor composition that has a sterility assurance level of 10⁻³ or less.

Other advantages and novel features of the present invention will become apparent from the following detailed description of various non-limiting embodiments of the invention when considered in conjunction with the accompanying figures. In cases where the present specification and a document incorporated by reference include conflicting and/or inconsistent disclosure, the present specification shall control.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
FIG. 1A is a schematic diagram of an exemplary sterilization system comprising a sterilization unit, according to some embodiments;
FIG. 1B is a schematic diagram of an exemplary sterilization system comprising a cascading water sterilizer, according to some embodiments;
FIG. 1C is a schematic diagram of an exemplary sterilization system comprising a cascading water sterilizer and a heater, according to some embodiments;
FIG. 2 is, according to some embodiments, a schematic diagram of an exemplary sterilization system comprising two sterilization units, two heaters, a chiller, a chiller pump reservoir, a deionized water pump reservoir, and two drying units;
FIG. 3A is a graph of percentage of available iodine over time for six povidone-iodine formulations sterilized at 110°C; and
FIG. 3B is a graph of percentage of available iodine over time for six povidone-iodine formulations sterilized at 115°C.

### DETAILED DESCRIPTION

Systems and methods for sterilizing iodophor compositions are described. In some cases, the iodophor composition comprises a complex comprising iodine and a polymer (i.e., an iodine-polymer complex). The iodine-polymer complex may be polyvinylpyrrolidone iodine (also referred to as "povidone-iodine" or "PVP-I") or iodine povacrylex. Methods are described for heating an initial iodophor composition to produce a sterilized iodophor composition. The resulting sterilized iodophor composition may have a relatively high assurance of sterility (e.g., a sterility assurance level of 10⁻³ or less, 10⁻⁶ or less). In some cases, heating the initial iodophor composition comprises exposing at least a portion of a container containing the initial iodophor composition to a heated liquid. In certain cases, the heated liquid is part of a moving liquid stream (e.g., a cascading waterfall stream produced by a cascading waterfall sterilizer). According to some embodiments, it was surprisingly determined that heating an initial iodophor composition even to a relatively low maximum temperature (i.e., a "sterilization temperature") while maintaining the sterilization temperature for a relatively short amount of time (i.e., a "sterilization time") can still lead to a relatively high assurance of sterility using certain methods described herein. As a result, sterilization systems and methods described herein may advantageously preserve certain positive attributes of an initial iodophor composition (e.g., potency, pH, viscosity).

An iodophor composition generally refers to a composition comprising an iodine-containing complex, typically dissolved or suspended in a liquid solvent (e.g., water and/or an alcohol) or other fluent carrier, including but not limited to a cream, ointment, or gel (e.g., a hydrogel). In some instances, the iodine-containing complex is a complex comprising iodine and a polymer (i.e., an iodine-polymer complex). Non-limiting examples of suitable polymers include homopolymers and copolymers comprising a vinylpyrrolidone and/or an acrylate (e.g., methyl methacrylate, isooctyl acrylate). In certain embodiments, the iodophor composition comprises povidone-iodine. Povidone-iodine is a complex comprising polyvinylpyrrolidone (also referred to as "povidone" or "PVP") and iodine. Povidone-iodine generally has the following chemical structure, where m and n represent the number of repeat units of each indicated structure in the overall polymer: 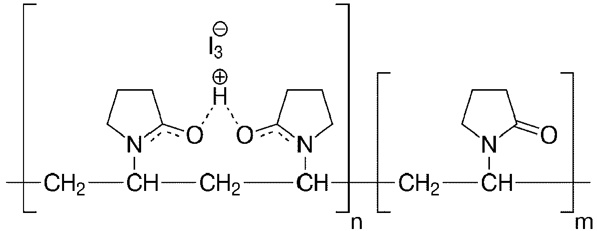 In certain embodiments, the iodophor composition comprises iodine povacrylex. Iodine povacrylex is an active ingredient in 3M's DuraPrep™ formulation (an iodine povacrylex and isopropyl alcohol solution available from the 3M Company of St. Paul, Minn.). Povacrylex is an acrylate copolymer. In certain embodiments, the iodophor composition comprises a complex comprising iodine (e.g., diiodine, sodium iodide) and poly(methyl methacrylate-co-N-vinylpyrrolidone-co-isooctyl acrylate). In some such embodiments, the iodine-polymer complex has the following chemical structure, where n1, n2, n3, and x represent the number of repeat units of each indicated structure in the overall polymer:

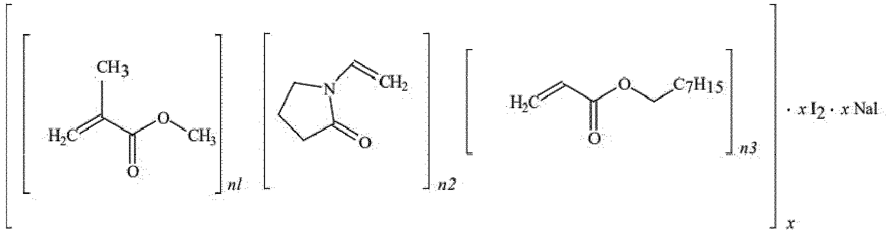

In some cases, an iodophor composition has antiseptic properties. In certain instances, an antiseptic iodophor composition may be topically applied to one or more locations on a patient (e.g., a human or animal) to reduce, treat, and/or prevent infection (e.g., by reducing microbial load). In some cases, an iodophor composition may be applied to intact skin of a patient. In some cases, an iodophor composition may be applied to, and/or in the vicinity of, a surgical incision, a wound, an ulcer, a cut, an abrasion, and/or a burn. In certain cases, an iodophor composition may be used as a preoperative disinfectant (i.e., to disinfect a surgical site prior to surgery), a surgical hand wash, and/or a general disinfectant. In some instances, an iodophor composition may be applied using a dispenser configured for topical application to a patient. Iodophor compositions described herein may be formulated as solutions, suspensions, emulsions, gels, creams, and/or ointments.

Without wishing to be bound by a particular theory, the mode of action of the antiseptic effect of an iodophor composition is believed to involve release of iodine (e.g., as iodine or triiodide) from the iodine-containing complex contained in the iodophor composition. In certain embodiments, an iodophor composition comprising povidone-iodine and/or iodine povacrylex releases iodine (e.g., as iodine or triiodide) over time, thereby providing an antiseptic effect. Iodine is generally considered an active antimicrobial agent that can kill various types of microorganisms (e.g., bacteria, viruses, fungi, protozoa). For example, iodine can kill microorganisms and/or reduce or inhibit their growth. In certain cases, iodine may kill transient skin flora and/or resident skin flora.

In some cases, it may be necessary, advantageous, or desirable to provide an iodophor composition in a sterilized form. For example, certain iodophor compositions may themselves be capable of supporting the growth of certain microorganisms or rapidly killing the microorganisms. In some instances, a relatively large number of microorganisms may be present in an iodophor composition. Sterilization of such an iodophor composition may advantageously reduce and/or prevent introduction of harmful microorganisms to a location on a patient where the iodophor composition is applied. This may be particularly beneficial when the iodophor composition is applied to, and/or in the vicinity of, a surgical incision, a wound, an ulcer, a cut, an abrasion, and/or a burn to prevent the patient from becoming infected with any microorganisms that may be present in the iodophor composition.

However, many conventional sterilization methods can negatively affect the effectiveness and/or certain other beneficial properties of an iodophor composition. For example, sterilization using exposure to gamma radiation can induce degradation and/or gelation of povidone-iodine, thereby destroying or reducing its effectiveness. In addition, it has been recognized that prolonged heating of an iodophor composition and/or heating of an iodophor composition at high temperatures can cause substantial degradation of the iodophor composition. For example, prolonged heating of an iodophor composition, particularly at high temperatures, can alter the pH, viscosity, color, solubility, and/or concentration of available iodine of the iodophor composition to a degree sufficient to reduce the efficacy of the iodophor composition, increase the difficulty of applying the iodophor composition to a patient, and/or reduce the safety of the iodophor composition. For such reasons, heat sterilization is not typically used for iodophor compositions formulated as antiseptic agents.

Surprisingly, the present inventors have determined within the context of compositions and methods described herein that an iodophor composition may be effectively sterilized by heating the iodophor composition to a relatively low sterilization temperature for a relatively short sterilization time period. According to certain sterilization systems and methods described herein, heating the iodophor composition to a relatively low sterilization temperature for a relatively short sterilization time period may achieve a relatively high assurance of sterility (e.g., a sterility assurance level of 10⁻³ or less, 10⁻⁶ or less). In some cases, sterilization of an iodophor composition according to sterilization systems and methods described herein can also reduce or minimize adverse impacts on positive attributes of the iodophor composition, including but not limited to pH, viscosity, appearance, solubility, and concentration of available iodine that can otherwise result from heat treatment.

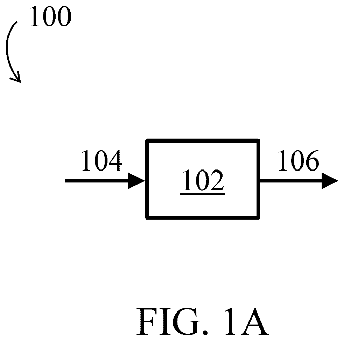

FIG. 1A illustrates a schematic diagram of an exemplary system for sterilizing an iodophor composition. As shown in FIG. 1A, sterilization system 100 comprises sterilization unit 102. Sterilization unit 102 may comprise any device configured to heat an object. In some embodiments, sterilization unit 102 is configured to perform moist heat sterilization. Examples of suitable sterilization units include, but are not limited to, a cascading water sterilizer and an autoclave. In certain preferred embodiments, sterilization unit 102 is a cascading water sterilizer. A non-limiting example of a suitable cascading water sterilizer is a SWS sterilizer, Model No. SWS-14.12.26, manufactured by Bosch Packaging Technology.

In operation, sterilization unit 102 receives initial iodophor composition 104. Sterilization unit 102 then heats initial iodophor composition 104 to a sterilization temperature for a sterilization time period. The "sterilization temperature" refers to the temperature or temperature range that the iodophor composition reaches and maintains during the sterilization time period. The "sterilization time" or "sterilization time period" refers to the length of time at which the iodophor composition is heated to the sterilization temperature. The sterilization temperature sets the maximum temperature that the iodophor composition can reach during the sterilization time period. Exposure of initial iodophor composition 104 to the sterilization temperature is terminated upon expiration of the sterilization time period. In some embodiments, initial iodophor composition 104 is heated in sterilization unit 102 to a relatively low sterilization temperature for a relatively short sterilization time period. Heating initial iodophor composition 104 to the sterilization temperature for the sterilization time period produces sterilized iodophor composition 106.

In some embodiments, the initial iodophor composition is heated to a relatively low sterilization temperature. In certain embodiments, the sterilization temperature of the iodophor composition is 121°C or less, 115°C or less, 110°C or less, 100°C or less, 95°C or less, 90°C or less, 85°C or less, 80°C or less, 75°C or less, 70°C or less, or 65°C or less. In certain embodiments, the sterilization temperature is in a range from 65°C to 85°C, 65°C to 100°C, 65°C to 110°C, 65°C to 115°C, 65°C to 121°C, 75°C to 85°C, 75°C to 100°C, 75°C to 110°C, 75°C to 115°C, 75°C to 121°C, 85°C to 100°C, 85°C to 110°C, 85°C to 115°C, 85°C to 121°C, 90°C to 100°C, 90°C to 110°C, 90°C to 115°C, 90°C to 121°C, 95°C to 110°C, 95°C to 115°C, 95°C to 121°C, 100°C to 110°C, 100°C to 115°C, 100°C to 121°C, 110°C to 115°C, 110°C to 121°C, or 115°C to 121°C. The sterilization temperature of the iodophor composition may be measured according to any method known in the art. In some cases, the temperature of the iodophor composition may not be measured directly (e.g., in certain embodiments when the iodophor composition is contained in a sealed ampoule, it may be difficult or inconvenient to measure the temperature directly), but rather may be determined or inferred based on a measured temperature of a fluid heating a container containing the iodophor composition and an appropriate correlation between the heating fluid temperature and exposure time to the temperature of the iodophor composition in such container. Those skilled in the art know how to experimentally and/or theoretically determine such correlation. For example, a temperature measuring device can be provided that measures the temperature of recirculating water within a sterilization unit. In some such cases, experiments and/or standard heat transfer calculations have been previously conducted to establish a correlation between the temperature of an iodophor composition within a container being treated (e.g., an ampoule) in a sterilization unit and the temperature of recirculating water as a function of exposure time. In certain cases, conditions and/or containers are chosen so that the temperature of the iodophor composition within the container equilibrates such that it becomes closely aligned with the temperature of recirculating water in the sterilization unit quickly to minimize treatment time. In some instances, in each sample or in a reference of the iodophor composition being treated, the temperature of the iodophor composition may be measured directly, for example with a temperature measuring device at least partially immersed in the iodophor composition undergoing sterilization.

In some embodiments, the initial iodophor composition is heated to the sterilization temperature for a relatively short sterilization time period. As noted above, the "sterilization time" or "sterilization time period" refers to the length of time at which the iodophor composition is heated to the sterilization temperature. The "sterilization time period" does not include the time it takes for the iodophor composition to reach the sterilization temperature from a starting temperature (i.e., it does not include "ramp up" time). The "sterilization time period" also does not include the time it takes for the iodophor composition to return to the starting temperature from the sterilization temperature (i.e., it does not include "cool down" time). In some embodiments, the sterilization time period is 20 minutes or less, 15 minutes or less, 12 minutes or less, 10 minutes or less, 6 minutes or less, 5 minutes or less, 4 minutes or less, 3 minutes or less, 2 minutes or less, 1.5 minutes or less, or 1 minute or less. In some embodiments, the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes.

In some embodiments, the sterilization temperature is relatively low and the sterilization time period is relatively short. In certain instances, the sterilization temperature is in a range from 65°C to 85°C, 65°C to 100°C, 65°C to 110°C, 65°C to 115°C, 65°C to 121°C, 75°C to 85°C, 75°C to 100°C, 75°C to 110°C, 75°C to 115°C, 75°C to 121°C, 85°C to 100°C, 85°C to 110°C, 85°C to 115°C, 85°C to 121°C, 90°C to 100°C, 90°C to 110°C, 90°C to 115°C, 90°C to 121°C, 95°C to 110°C, 95°C to 115°C, 95°C to 121°C, 100°C to 110°C, 100°C to 115°C, 100°C to 121°C, 110°C to 115°C, 110°C to 121°C, or 115°C to 121°C, and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes.

In certain instances, the sterilization temperature is about 95°C and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes. In certain instances, the sterilization temperature is about 100°C and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes. In certain instances, the sterilization temperature is about 105°C and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes. In certain instances, the sterilization temperature period is about 110°C and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes. In certain instances, the sterilization temperature is about 115°C and the sterilization time period is in a range from 1 minute to 3 minutes, 1 minute to 6 minutes, 1 minute to 10 minutes, 1 minute to 12 minutes, 1 minute to 15 minutes, 1 minute to 20 minutes, 1.5 minutes to 3 minutes, 1.5 minutes to 6 minutes, 1.5 minutes to 10 minutes, 1.5 minutes to 12 minutes, 1.5 minutes to 15 minutes, 1.5 minutes to 20 minutes, 3 minutes to 6 minutes, 3 minutes to 10 minutes, 3 minutes to 12 minutes, 3 minutes to 15 minutes, 3 minutes to 20 minutes, 6 minutes to 10 minutes, 6 minutes to 12 minutes, 6 minutes to 15 minutes, 6 minutes to 20 minutes, 10 minutes to 12 minutes, 10 minutes to 15 minutes, 10 minutes to 20 minutes, 12 minutes to 15 minutes, 12 minutes to 20 minutes, or 15 minutes to 20 minutes.

In some embodiments, heating an initial iodophor composition to a sterilization temperature for a sterilization time period produces a sterilized iodophor composition having a relatively high assurance of sterility. The sterility assurance level ("SAL") of a product provides a measure of the probability that the product will remain nonsterile after undergoing a sterilization process. As illustrative examples, an SAL of 10⁻³ means that there is a 1 in 1,000 chance of a viable microorganism being present in a sterilized product, and an SAL of 10⁻⁶ means that there is a 1 in 1,000,000 chance of a viable microorganism being present in a sterilized product. An SAL of 10⁻⁶ or less encompasses SAL values of 10⁻⁶, 10⁻⁷, 10⁻⁸, 10⁻⁹, etc. In some embodiments, the sterilized iodophor composition has an SAL of 10⁻³ or less, 10⁻⁴ or less, 10⁻⁵ or less, 10⁻⁶ or less, 10⁻⁷ or less, 10⁻⁸ or less, 10⁻⁹ or less, 10⁻¹⁰ or less, 10⁻¹¹ or less, or 10⁻¹² or less. In certain embodiments, the sterilized iodophor composition has an SAL of 10⁻⁴ to 10⁻³, 10⁻⁵ to 10⁻³, 10⁻⁶ to 10⁻³, 10⁻⁷ to 10⁻³, 10⁻⁸ to 10⁻³, 10⁻⁹ to 10⁻³, 10⁻¹⁰ to 10⁻³, 10⁻¹¹ to 10⁻³, 10⁻¹² to 10⁻³, 10⁻⁵ to 10⁻⁴, 10⁻⁶ to 10⁻⁴, 10⁻⁷ to 10⁻⁴, 10⁻⁸ to 10⁻⁴, 10⁻⁹ to 10⁻⁴, 10⁻¹⁰ to 10⁻⁴, 10⁻¹¹ to 10⁻⁴, 10⁻¹² to 10⁻⁴, 10⁻⁶ to 10⁻⁵, 10⁻⁷ to 10⁻⁵, 10⁻⁸ to 10⁻⁵, 10⁻⁹ to 10⁻⁵, 10⁻¹⁰ to 10⁻⁵, 10⁻¹¹ to 10⁻⁵, 10⁻¹² to 10⁻⁵, 10⁻⁷ to 10⁻⁶, 10⁻⁸ to 10⁻⁶, 10⁻⁹ to 10⁻⁶, 10⁻¹⁰ to 10⁻⁶, 10⁻¹¹ to 10⁻⁶, 10⁻¹² to 10⁻⁶, 10⁻⁸ to 10⁻⁷, 10⁻⁹ to 10⁻⁷, 10⁻¹⁰ to 10⁻⁷, 10⁻¹¹ to 10⁻⁷, 10⁻¹² to 10⁻⁷, 10⁻⁹ to 10⁻⁸, 10⁻¹⁰ to 10⁻⁸, 10⁻¹¹ to 10⁻⁸, 10⁻¹² to 10⁻⁸, 10⁻¹⁰ to 10⁻⁹, 10⁻¹¹ to 10⁻⁹, 10⁻¹² to 10⁻⁹, 10⁻¹¹ to 10⁻¹⁰, 10⁻¹² to 10⁻¹⁰, or 10⁻¹¹ to 10⁻¹². An SAL of 10⁻³ or less may be suitable for a composition applied topically to intact skin. A standard approved by the American National Standards Institute (ANSI) and the Association for the Advancement of Medical Instrumentation (AAMI), ANSI/AAMI ST67:2011/(R)2017, *Sterilization of Health Care Products-Requirements and Guidance for Selecting a Sterility Assurance Level (SAL) for Products Labeled "Sterile,* " has specified that an SAL of 10⁻³ or less shall be used for topical products that contact intact skin or mucous membranes and are not intended to come into contact with breached skin or compromised tissue. An SAL of 10⁻⁶ or less may be suitable for a composition contacting a surgical incision, a wound, an ulcer, a cut, an abrasion, and/or a burn. The ANSI/AAMI ST67:2011(R)2017 standard has specified that an SAL of 10⁻⁶ or less shall be used for products intended to come into contact with breached skin or compromised tissue (i.e., tissue that has lost its natural barrier integrity or is damaged or injured).

In some cases, the sterilization time and/or sterilization temperature required to achieve an SAL may be calculated from one or more "D-values." The term D-value has its ordinary meaning as used in microbiology. Specifically, it refers to the time required under specific sterilization conditions (e.g., exposure to a sterilization temperature) to kill 90% (i.e., 1 log) of a microorganism being studied (sometimes referred to as a "challenge microorganism"). A non-limiting example of a suitable challenge organism is *Geobacillus stearothermophilus.* As an illustrative example, the time required to achieve an SAL of 10⁻⁶ at a certain temperature may be calculated by multiplying the D-value at that temperature by 6 (i.e., to obtain the time required to obtain a 6-log reduction in counts of the challenge microorganism).

In some embodiments, heating an initial iodophor composition comprises exposing at least a portion of a container containing the initial iodophor composition to a heated liquid. Non-limiting examples of suitable liquids include water, aqueous solutions, alcohols, organic solvents, and oils. In certain embodiments, the heated liquid is water.

In some embodiments, the heated liquid is part of one or more moving liquid streams. In certain cases, heating an initial iodophor composition comprises contacting at least a portion of a container containing the initial iodophor composition with one or more moving liquid streams comprising a heated liquid. In some embodiments, the one or more moving liquid streams comprising a heated liquid comprise one or more cascading waterfall streams produced by a cascading water sterilizer. An exemplary cascading water sterilizer is described in U.S. Patent No. 9,511,156, by Degala et al., filed June 30, 2015, and entitled "Systems, Methods, and Devices for Sterilizing Antiseptic Solutions," the contents of which are incorporated herein by reference in their entirety for all purposes. In some embodiments, the heated liquid is part of a liquid bath. In certain embodiments, the liquid bath comprises one or more agitating elements (e.g., an agitator, a magnetic stir bar). In some cases, heating the initial iodophor composition comprises immersing at least a portion of a container containing the initial iodophor composition in a liquid bath.

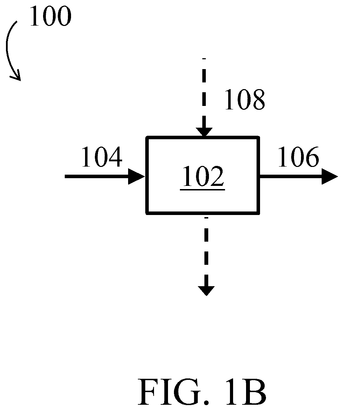

In some embodiments, a sterilization system comprises a cascading water sterilizer. FIG. 1B shows a schematic diagram of exemplary sterilization system 100 comprising cascading water sterilizer 102. In certain embodiments, cascading water sterilizer 102 comprises a chamber. The chamber may have any suitable shape and volume. In some embodiments, the chamber is coupled to a water dispensing device configured to create one or more cascading waterfall streams flowing through the chamber. In some embodiments, the water dispensing device is configured to create one or more cascading waterfall streams flowing from a top portion of the chamber to a bottom portion of the chamber. Examples of suitable water dispensing devices include, but are not limited to, a perforated sheet (e.g., a sheet comprising a plurality of holes) and one or more spray nozzles.

In operation, according to some embodiments, one or more containers containing initial iodophor composition 104 may be positioned within the chamber of cascading water sterilizer 102. In the chamber, water stream 108 may be directed to flow as a cascading waterfall from the top of the chamber to the bottom of the chamber. The cascading waterfall may be heated to a relatively high temperature (i.e., a temperature sufficient to raise the temperature of the initial iodophor composition in the container to the desired sterilization temperature). As water stream 108 flows from the top to the bottom of the chamber, it contacts at least a portion of the one or more containers containing initial iodophor composition 104, and heats the one or more containers containing initial iodophor composition 104. Conditions are chosen so that contact between water stream 108 and the one or more containers containing initial iodophor composition 104 can heat the initial iodophor composition 104 to the sterilization temperature for the sterilization time period to produce sterilized iodophor composition 106. In some embodiments, at least a portion of water stream 108 exits the bottom of the chamber of cascading water sterilizer 102 and is recirculated to the top of the chamber. In other embodiments, water stream 108 exits the bottom of the chamber of cascading water sterilizer 102 and is not recirculated.

In some embodiments, after completion of the sterilization time period, the chamber of cascading water sterilizer 102 is drained of water, and the exterior surfaces of the one or more containers containing sterilized iodophor composition 106 are dried. In some cases, the one or more containers are dried (e.g., exposed to heated, dehumidified air) within the chamber of cascading water sterilizer 102. In some cases, the one or more containers are transferred to a separate drying unit for drying.

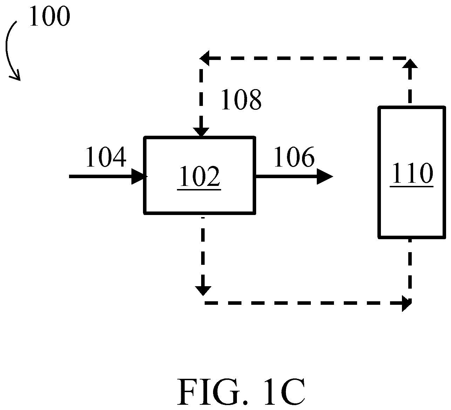

In some embodiments, a sterilization system (e.g., comprising a cascading water sterilizer) further comprises a heater configured to heat the liquid stream. Examples of suitable heaters include, but are not limited to, a heat exchanger, a boiler, an electric heater, and a heat pump. FIG. 1C shows a schematic diagram of an exemplary sterilization system 100 comprising cascading water sterilizer 102 and heater 110. As shown in FIG. 1C, cascading water sterilizer 102 is in fluidic communication with heater 110.

In some embodiments, at least a portion of water stream 108 exits through the bottom of the chamber of cascading water sterilizer 102 and is directed to flow through heater 110 and is subsequently returned to the top of the chamber of cascading water sterilizer 102.

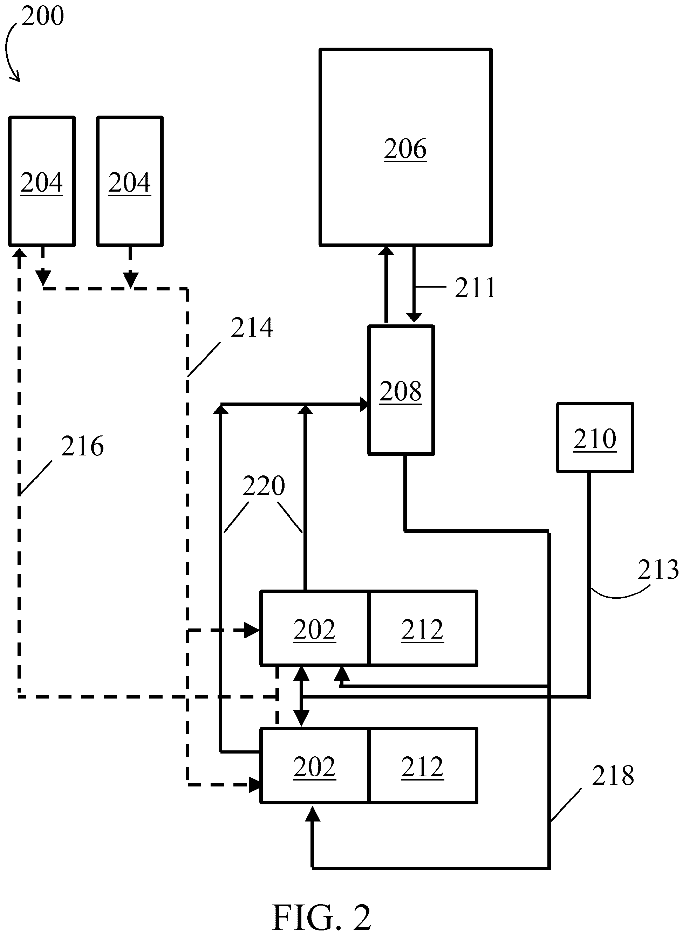

In some embodiments, a sterilization system comprises one or more additional components. For example, FIG. 2 shows a schematic diagram of an exemplary sterilization system 200 comprising two sterilization units 202 (e.g., cascading water sterilizers), two heaters 204, chiller 206, chiller pump reservoir 208, deionized water pump reservoir 210, and two drying units 212. In certain embodiments, heaters 204 are boilers.

In operation, a plurality of containers are filled with an iodophor composition and loaded into the chambers of sterilization units 202. Once the containers are placed in sterilization units 202 and the sterilization process is initiated, deionized water from deionized water pump reservoir 210 is pumped through input line 213 into the chambers of sterilization units 202. The deionized water is then continuously circulated within each chamber such that the deionized water falls over the containers.

As the deionized water is circulating within the chambers of sterilization units 202, steam from heaters 204 flows into sterilization units 202 via input line 214. The steam passes through a heat exchanger, and heat is transferred from the steam to the deionized water. After being cooled in the heat exchanger, steam condensate exits the heat exchanger and returns to heaters 204 via return line 216. The heat exchange cycle continues until the temperature of the circulating deionized water is determined to be at an appropriate temperature. The sterilization system may precisely control the input of steam from heaters 204 to maintain the appropriate sterilization conditions (e.g., by actuating the appropriate valves). During this heat exchange process, the deionized water continues to circulate within the chamber and fall over the containers, thereby heating the iodophor composition.

Once the sterilization time period has ended, a cooling process may be initiated. During the cooling process, water chilled by chiller 206 flows to chiller pump reservoir 208 via input and return lines 211. The chilled water travels from chiller pump reservoir 208 to sterilization units 202 via input line 218. Heat is then transferred from deionized water to the chilled water, thereby cooling the deionized water and heating the chilled water. During this cooling heat exchange process, the deionized water continues to circulate within the chamber and fall over the containers, thereby cooling the iodophor composition. Following the heat exchange, the chilled water returns to chiller pump reservoir 208 and chiller 206 via lines 220 and 211. The cooling process continues until the deionized water in the chamber has reached the desired temperature (e.g., room temperature).

Upon completion of the cooling process, the deionized water is drained from the chambers of sterilization units 202, and the containers are transferred to drying units 212. Drying units 212 may be used to dry the exterior surfaces of the containers. In certain embodiments, drying units 212 comprise a dehumidifier. In some instances, drying units 212 may employ heated, dehumidified air to dry the containers.

In some cases, a computer control system may be used to control sterilization system 200. In certain instances, the computer control system may include a processor programmed to provide the appropriate operating parameters (e.g., amount of deionized water circulated in the sterilization unit, sterilization temperature, sterilization time period) for particular iodophor compositions being sterilized.

In some embodiments, a sterilization system comprises a plurality of sterilization units. In certain cases, a sterilization system comprises at least 2, at least 5, or at least 10 sterilization units. In some embodiments, the number of sterilization units in a sterilization system is in a range from 1 to 2, 1 to 5, 1 to 10, 2 to 5, 2 to 10, or 5 to 10. In some embodiments, a sterilization system comprises a plurality of heating units and/or a plurality of cooling units.

In some embodiments, a method of sterilizing an iodophor composition comprises providing one or more containers containing an initial iodophor composition. The one or more containers may be any containers suitable for containing a volume of a liquid. Non-limiting examples of suitable containers include ampoules, jars, bottles, tubes, and pouches. In certain cases, the one or more containers are configured to stand vertically upright. In some embodiments, the one or more containers are sealed. The one or more containers may be made from any material suitable for containing an iodophor composition. Non-limiting examples of suitable materials for the one or more containers include glass, plastic, a polymer, and/or a metallic laminate. In certain embodiments, the one or more containers comprise a glass ampoule, a plastic ampoule, a plastic bottle, a plastic tube, a polymer pouch, or a metallic laminate pouch. The one or more containers may have any suitable size and shape. In certain embodiments, one or more containers may be sized to contain 0.67 mL, 0.75 mL, 1 mL, 1.5 mL, 3 mL, 5 mL, 10.5 mL, 13 mL, and/or 26 mL of a liquid. In certain embodiments, one or more containers are sized to contain an amount in a range from 0.67 mL to 1 mL, 0.67 mL to 3 mL, 0.67 mL to 5 mL, 0.67 mL to 10.5 mL, 0.67 mL to 13 mL, 0.67 mL to 26 mL, 0.75 mL to 1 mL, 0.75 mL to 3 mL, 0.75 mL to 5 mL, 0.75 mL to 10.5 mL, 0.75 mL to 13 mL, 0.75 mL to 26 mL, 1 mL to 3 mL, 1 mL to 5 mL, 1 mL to 10.5 mL, 1 mL to 13 mL, 1 mL to 26 mL, 3 mL to 10.5 mL, 3 mL to 13 mL, 3 mL to 26 mL, 10.5 mL to 13 mL, 10.5 mL to 26 mL, or 13 mL to 26 mL. In some cases, each of the one or more containers is configured to contain the same volume of liquid. In some cases, the one or more containers are configured to contain different volumes of liquid.

According to some embodiments, the initial iodophor composition has a relatively high concentration of an iodine-polymer complex (e.g., PVP-I, iodine povacrylex). In some cases, the concentration of a component may be expressed as a weight percentage (wt.%). The weight percentage of a component of a composition generally refers to the ratio of the mass of the component to the total mass of the composition, multiplied by 100. As an illustrative example, the weight percentage of an iodine-polymer complex (e.g., PVP-I, iodine povacrylex) in the initial iodophor composition can be determined by dividing the mass of the iodine-polymer complex (e.g., PVP-I, iodine povacrylex) by the total mass of the initial iodophor composition, and multiplying the quotient by 100. In some embodiments, the initial iodophor composition comprises the iodine-polymer complex (e.g., PVP-I, iodine povacrylex) in a concentration of at least 5.0 wt.%, at least 5.5 wt.%, at least 6.0 wt.%, at least 6.5 wt.%, at least 7.0 wt.%, at least 7.5 wt.%, at least 8.0 wt.%, at least 8.5 wt.%, at least 9.0 wt.%, at least 9.5 wt.%, or at least 10.0 wt.%. In certain embodiments, the initial iodophor composition comprises the iodine-polymer complex (e.g., PVP-I, iodine povacrylex) in a concentration in a range from 5.0 wt.% to 6.0 wt.%, 5.0 wt.% to 7.0 wt.%, 5.0 wt.% to 8.0 wt.%, 5.0 wt.% to 9.0 wt.%, 5.0 wt.% to 10.0 wt.%, 6.0 wt.% to 7.0 wt.%, 6.0 wt.% to 8.0 wt.%, 6.0 wt.% to 9.0 wt.%, 6.0 wt.% to 10.0 wt.%, 7.0 wt.% to 8.0 wt.%, 7.0 wt.% to 9.0 wt.%, 7.0 wt.% to 10.0 wt.%, 8.0 wt.% to 9.0 wt.%, 8.0 wt.% to 10.0 wt.%, or 9.0 wt.% to 10.0 wt.%.

In some embodiments, the initial iodophor composition has a relatively high concentration of available iodine (also referred to as percentage of available iodine). In certain embodiments, the initial iodophor composition comprises a concentration of available iodine of at least 0.30 wt.%, at least 0.40 wt.%, at least 0.50 wt.%, at least 0.55 wt.%, at least 0.60 wt.%, at least 0.65 wt.%, at least 0.70 wt.%, at least 0.75 wt.%, at least 0.80 wt.%, at least 0.85 wt.%, at least 0.90 wt.%, at least 0.95 wt.%, at least 1.0 wt.%, at least 1.2 wt.%, at least 1.3 wt.%, at least 1.4 wt.%, or at least 1.5 wt.%. In some embodiments, the initial iodophor composition comprises a concentration of available iodine in a range from 0.30 wt.% to 0.50 wt.%, 0.30 wt.% to 0.60 wt.%, 0.30 wt.% to 0.70 wt.%, 0.30 wt.% to 0.80 wt.%, 0.30 wt.% to 0.90 wt.%, 0.30 wt.% to 1.0 wt.%, 0.30 wt.% to 1.1 wt.%, 0.30% to 1.2%, 0.30 wt.% to 1.3 wt.%, 0.30 wt.% to 1.4 wt.%, 0.30 wt.% to 1.5 wt.%, 0.40 wt.% to 0.60 wt.%, 0.40 wt.% to 0.70 wt.%, 0.40 wt.% to 0.80 wt.%, 0.40 wt.% to 0.90 wt.%, 0.40 wt.% to 1.0 wt.%, 0.40 wt.% to 1.1 wt.%, 0.40% to 1.2%, 0.40 wt.% to 1.3 wt.%, 0.40 wt.% to 1.4 wt.%, 0.40 wt.% to 1.5 wt.%, 0.50 wt.% to 0.70 wt.%, 0.50 wt.% to 0.80 wt.%, 0.50 wt.% to 0.90 wt.%, 0.50 wt.% to 1.0 wt.%, 0.50 wt.% to 1.1 wt.%, 0.50% to 1.2%, 0.50 wt.% to 1.3 wt.%, 0.50 wt.% to 1.4 wt.%, 0.50 wt.% to 1.5 wt.%, 0.60 wt.% to 0.80 wt.%, 0.60 wt.% to 0.90 wt.%, 0.60 wt.% to 1.0 wt.%, 0.60 wt.% to 1.1 wt.%, 0.60% to 1.2%, 0.60 wt.% to 1.3 wt.%, 0.60 wt.% to 1.4 wt.%, 0.60 wt.% to 1.5 wt.%, 0.70 wt.% to 0.90 wt.%, 0.70 wt.% to 1.0 wt.%, 0.70 wt.% to 1.1 wt.%, 0.70% to 1.2%, 0.70 wt.% to 1.3 wt.%, 0.70 wt.% to 1.4 wt.%, 0.70 wt.% to 1.5 wt.%, 0.80 wt.% to 1.0 wt.%, 0.80 wt.% to 1.1 wt.%, 0.80% to 1.2%, 0.80 wt.% to 1.3 wt.%, 0.80 wt.% to 1.4 wt.%, 0.80 wt.% to 1.5 wt.%, 0.90 wt.% to 1.1 wt.%, 0.90% to 1.2%, 0.90 wt.% to 1.3 wt.%, 0.90 wt.% to 1.4 wt.%, 0.90 wt.% to 1.5 wt.%, 1.0% to 1.2%, 1.0 wt.% to 1.3 wt.%, 1.0 wt.% to 1.4 wt.%, 1.0 wt.% to 1.5 wt.%, 1.1 wt.% to 1.5 wt.%, 1.2 wt.% to 1.5 wt.%, 1.3 wt.% to 1.5 wt.%, or 1.4 wt.% to 1.5 wt.%. The concentration of available iodine in an iodophor composition may be determined according to any method known in the art. For example, the concentration of available iodine in an iodophor composition may be measured by titrimetry using sodium thiosulfate.

In some embodiments, the initial iodophor composition comprises an iodine-polymer complex (e.g., PVP-I, iodine povacrylex) dissolved and/or suspended in a solvent. Non-limiting examples of suitable solvents include water, alcohols, acetone, polyethylene glycol, glycerol, and combinations thereof. In some embodiments, the initial and/or sterilized iodophor composition comprises a solvent in a concentration of at least 50 wt.%, at least 60 wt.%, at least 70 wt.%, at least 80 wt.%, at least 90 wt.%, or at least 95 wt.%. In some embodiments, the initial and/or sterilized iodophor composition comprises a solvent in a concentration in a range from 50 wt.% to 80 wt.%, 50 wt.% to 90 wt.%, 50 wt.% to 95 wt.%, 60 wt.% to 80 wt.%, 60 wt.% to 90 wt.%, 60 wt.% to 95 wt.%, 70 to 90 wt.%, 70 to 95 wt.%, or 80 wt.% to 95 wt.%.

In some embodiments, the initial iodophor composition comprises water and has an acidic or neutral pH. In some embodiments, the initial iodophor composition has a pH of at least 1.5, at least 2.0, at least 2.5, at least 3.0, at least 3.5, at least 4.0, at least 4.5, at least 5.0, at least 5.5, at least 6.0, at least 6.5, or at least 7.0. In some embodiments, the initial iodophor composition has a pH of 7.0 or less, 6.5 or less, 6.0 or less, 5.5 or less, 5.0 or less, 4.5 or less, 4.0 or less, 3.5 or less, 3.0 or less, 2.5 or less, 2.0 or less, or 1.5 or less. In some embodiments, the initial iodophor composition has a pH in a range from 1.5 to 3.0, 1.5 to 4.0, 1.5 to 5.0, 1.5 to 6.0, 1.5 to 6.5, 1.5 to 7.0, 2.0 to 4.0, 2.0 to 5.0, 2.0 to 5.5, 2.0 to 6.0, 2.0 to 6.5, 2.0 to 7.0, 3.0 to 5.0, 3.0 to 6.0, 3.0 to 6.5, 3.0 to 7.0, 4.0 to 6.0, 4.0 to 6.5, 4.0 to 7.0, 5.0 to 6.0, 5.0 to 6.5, 5.0 to 7.0, or 6.0 to 7.0.

In some embodiments, the initial iodophor composition has a viscosity that facilitates application of the initial iodophor composition to one or more locations on a patient. In some embodiments, the initial iodophor composition has a viscosity at ambient temperature (i.e., about 20°C) of at least 500 centipoise (cP), at least 600 cP, at least 700 cP, at least 800 cP, at least 900 cP, at least 1000 cP, at least 1500 cP, at least 2000 cP, at least 2500 cP, or at least 3000 cP. In some embodiments, the initial iodophor composition has a viscosity at ambient temperature of 3000 cP or less, 2500 cP or less, 2000 cP or less, 1500 cP or less, 1000 cP or less, or 500 cP or less. In some embodiments, the initial iodophor composition has a viscosity at ambient temperature in a range from 500 cP to 1000 cP, 500 cP to 1500 cP, 500 cP to 2000 cP, 500 cP to 2500 cP, 500 cP to 3000 cP, 1000 cP to 1500 cP, 1000 cP to 2000 cP, 1000 cP to 2500 cP, 1000 cP to 3000 cP, 1500 cP to 2000 cP, 1500 cP to 2500 cP, 1500 cP to 3000 cP, 2000 cP to 2500 cP, or 2000 cP to 3000 cP. The viscosity of the initial iodophor composition at ambient temperature may be measured according to any method known in the art. For example, the viscosity of the initial iodophor composition at ambient temperature may be measured using a rotational viscometer.

The initial iodophor composition may have any form suitable for application to a location on a patient. In some embodiments, the initial iodophor composition is a gel, solution, suspension, emulsion, cream, ointment, lotion, paste, medicated stick, and/or balm. In some embodiments, the initial iodophor composition is configured to facilitate topical application on a location on a patient. The initial iodophor composition may have any suitable appearance. In some embodiments, the initial iodophor composition does not comprise any solid phase components. In certain embodiments, for example, the initial iodophor composition does not comprise any visible precipitate or solid particles.

According to some embodiments, the sterilized iodophor composition maintains certain advantageous properties of the initial iodophor composition. In some cases, sterilization systems, methods, and conditions described herein can reduce and/or minimize certain negative impacts of sterilization on one or more properties of the initial iodophor composition important to its effectiveness or suitability for its intended use. In some embodiments, for example, the sterilized iodophor composition retains a relatively high concentration of an iodine-polymer complex (e.g., PVP-I, iodine povacrylex). In certain embodiments, the sterilized iodophor composition comprises an iodine-polymer complex (e.g., PVP-I, iodine povacrylex) in a concentration of at least 5.0 wt.%, at least 5.5 wt.%, at least 6.0 wt.%, at least 6.5 wt.%, at least 7.0 wt.%, at least 7.5 wt.%, at least 8.0 wt.%, at least 8.5 wt.%, at least 9.0 wt.%, at least 9.5 wt.%, or at least 10.0 wt.%. In certain embodiments, the sterilized iodophor composition comprises an iodine-polymer complex (e.g., PVP-I, iodine povacrylex) in a concentration in a range from 5.0 wt.% to 6.0 wt.%, 5.0 wt.% to 7.0 wt.%, 5.0 wt.% to 8.0 wt.%, 5.0 wt.% to 9.0 wt.%, 5.0 wt.% to 10.0 wt.%, 6.0 wt.% to 7.0 wt.%, 6.0 wt.% to 8.0 wt.%, 6.0 wt.% to 9.0 wt.%, 6.0 wt.% to 10.0 wt.%, 7.0 wt.% to 8.0 wt.%, 7.0 wt.% to 9.0 wt.%, 7.0 wt.% to 10.0 wt.%, 8.0 wt.% to 9.0 wt.%, 8.0 wt.% to 10.0 wt.%, or 9.0 wt.% to 10.0 wt.%.

In some embodiments, any decrease in concentration of an iodine-polymer complex (e.g., PVP-I, iodine povacrylex) resulting from sterilization is relatively low. In some embodiments, the percent difference between a first iodine-polymer complex (e.g., PVP-I, iodine povacrylex) concentration of the initial iodophor composition and a second iodine-polymer complex (e.g., PVP-I, iodine povacrylex) concentration of the sterilized iodophor composition is 8% or less, 7% or less, 6% or less, 5% or less, 4% or less, 3% or less, 2% or less, 1% or less, 0.5% or less, or 0.1% or less. In some embodiments, the percent difference between a first iodine-polymer complex (e.g., PVP-I, iodine povacrylex) concentration of the initial iodophor composition and a second iodine-polymer complex (e.g., PVP-I, iodine povacrylex) concentration of the sterilized iodophor composition is in a range from 0.1% to 0.5%, 0.1% to 1%, 0.1% to 2%, 0.1% to 5%, 0.1% to 6%, 0.1% to 7%, 0.1% to 8%, 0.5% to 1%, 0.5% to 2%, 0.5% to 5%, 0.5% to 6%, 0.5% to 7%, 0.5% to 8%, 1% to 2%, 1% to 5%, 1% to 6%, 1% to 7%, 1% to 8%, 2% to 5%, 2% to 6%, 2% to 7%, 2% to 8%, 3% to 5%, 3% to 6%, 3% to 7%, 3% to 8%, 4% to 6%, 4% to 7%, 4% to 8%, 5% to 7%, 5% to 8%, or 6% to 8%.

In some embodiments, the absolute value of the difference between a first iodine-polymer complex (e.g., PVP-I, iodine povacrylex) concentration of the initial iodophor composition and a second iodine-polymer complex (e.g., PVP-I, iodine povacrylex) concentration of the sterilized iodophor composition is 1 wt.% or less, 0.9 wt.% or less, 0.8 wt.% or less, 0.7 wt.% or less, 0.6 wt.% or less, 0.5 wt.% or less, 0.4 wt.% or less, 0.3 wt.% or less, 0.2 wt.% or less, 0.1 wt.% or less, 0.05 wt.% or less, 0.02 wt.% or less, or 0.01 wt.% or less. In some embodiments, the absolute value of the difference between a first iodine-polymer complex (e.g., PVP-I, iodine povacrylex) concentration of the initial iodophor composition and a second iodine-polymer complex (e.g., PVP-I, iodine povacrylex) concentration of the sterilized iodophor composition is in a range from 0.01 wt.% to 0.05 wt.%, 0.01 wt.% to 0.1 wt.%, 0.01 wt.% to 0.2 wt.%, 0.01 wt.% to 0.3 wt.%, 0.01 wt.% to 0.4 wt.%, 0.01 wt.% to 0.5 wt.%, 0.01 wt.% to 0.6 wt.%, 0.01 wt.% to 0.7 wt.%, 0.01 wt.% to 0.8 wt.%, 0.01 wt.% to 0.9 wt.%, 0.01 wt.% to 1 wt.%, 0.05 wt.% to 0.1 wt.%, 0.05 wt.% to 0.5 wt.%, 0.05 wt.% to 1 wt.%, 0.1 wt.% to 0.5 wt.%, 0.1 wt.% to 1 wt.%, or 0.5 wt.% to 1 wt.%.

In some embodiments, the sterilized iodophor composition has a relatively high concentration of available iodine. In certain embodiments, the sterilized iodophor composition comprises a concentration of available iodine of at least 0.30 wt.%, at least 0.40 wt.%, at least 0.50 wt.%, at least 0.55 wt.%, at least 0.60 wt.%, at least 0.65 wt.%, at least 0.70 wt.%, at least 0.75 wt.%, at least 0.80 wt.%, at least 0.85 wt.%, at least 0.90 wt.%, at least 0.95 wt.%, at least 1.0 wt.%, at least 1.2 wt.%, at least 1.3 wt.%, at least 1.4 wt.%, or at least 1.5 wt.%. In some embodiments, the sterilized iodophor composition comprises a concentration of available iodine in a range from 0.30 wt.% to 0.50 wt.%, 0.30 wt.% to 0.60 wt.%, 0.30 wt.% to 0.70 wt.%, 0.30 wt.% to 0.80 wt.%, 0.30 wt.% to 0.90 wt.%, 0.30 wt.% to 1.0 wt.%, 0.30 wt.% to 1.1 wt.%, 0.30% to 1.2%, 0.30 wt.% to 1.3 wt.%, 0.30 wt.% to 1.4 wt.%, 0.30 wt.% to 1.5 wt.%, 0.40 wt.% to 0.60 wt.%, 0.40 wt.% to 0.70 wt.%, 0.40 wt.% to 0.80 wt.%, 0.40 wt.% to 0.90 wt.%, 0.40 wt.% to 1.0 wt.%, 0.40 wt.% to 1.1 wt.%, 0.40% to 1.2%, 0.40 wt.% to 1.3 wt.%, 0.40 wt.% to 1.4 wt.%, 0.40 wt.% to 1.5 wt.%, 0.50 wt.% to 0.70 wt.%, 0.50 wt.% to 0.80 wt.%, 0.50 wt.% to 0.90 wt.%, 0.50 wt.% to 1.0 wt.%, 0.50 wt.% to 1.1 wt.%, 0.50% to 1.2%, 0.50 wt.% to 1.3 wt.%, 0.50 wt.% to 1.4 wt.%, 0.50 wt.% to 1.5 wt.%, 0.60 wt.% to 0.80 wt.%, 0.60 wt.% to 0.90 wt.%, 0.60 wt.% to 1.0 wt.%, 0.60 wt.% to 1.1 wt.%, 0.60% to 1.2%, 0.60 wt.% to 1.3 wt.%, 0.60 wt.% to 1.4 wt.%, 0.60 wt.% to 1.5 wt.%, 0.70 wt.% to 0.90 wt.%, 0.70 wt.% to 1.0 wt.%, 0.70 wt.% to 1.1 wt.%, 0.70% to 1.2%, 0.70 wt.% to 1.3 wt.%, 0.70 wt.% to 1.4 wt.%, 0.70 wt.% to 1.5 wt.%, 0.80 wt.% to 1.0 wt.%, 0.80 wt.% to 1.1 wt.%, 0.80% to 1.2%, 0.80 wt.% to 1.3 wt.%, 0.80 wt.% to 1.4 wt.%, 0.80 wt.% to 1.5 wt.%, 0.90 wt.% to 1.1 wt.%, 0.90% to 1.2%, 0.90 wt.% to 1.3 wt.%, 0.90 wt.% to 1.4 wt.%, 0.90 wt.% to 1.5 wt.%, 1.0% to 1.2%, 1.0 wt.% to 1.3 wt.%, 1.0 wt.% to 1.4 wt.%, 1.0 wt.% to 1.5 wt.%, 1.1 wt.% to 1.5 wt.%, 1.2 wt.% to 1.5 wt.%, 1.3 wt.% to 1.5 wt.%, or 1.4 wt.% to 1.5 wt.%.

In some embodiments, any percentage decrease in concentration of available iodine is relatively low. In some embodiments, the percent difference between a first concentration of available iodine of the initial iodophor composition and a second concentration of available iodine of the sterilized iodophor composition is 8% or less, 7% or less, 6% or less, 5% or less, 4% or less, 3% or less, 2% or less, 1% or less, 0.5% or less, or 0.1% or less. In some embodiments, the percent difference between a first concentration of available iodine of the initial iodophor composition and a second concentration of available iodine of the sterilized iodophor composition is in a range from 0.1% to 0.5%, 0.1% to 1%, 0.1% to 2%, 0.1% to 5%, 0.1% to 6%, 0.1% to 7%, 0.1% to 8%, 0.5% to 1%, 0.5% to 2%, 0.5% to 5%, 0.5% to 6%, 0.5% to 7%, 0.5% to 8%, 1% to 2%, 1% to 5%, 1% to 6%, 1% to 7%, 1% to 8%, 2% to 5%, 2% to 6%, 2% to 7%, 2% to 8%, 3% to 5%, 3% to 6%, 3% to 7%, 3% to 8%, 4% to 6%, 4% to 7%, 4% to 8%, 5% to 7%, 5% to 8%, or 6% to 8%.

In some embodiments, the absolute value of the difference between a first concentration of available iodine of the initial iodophor composition and a second concentration of available iodine of the sterilized iodophor composition is 1 wt.% or less, 0.9 wt.% or less, 0.8 wt.% or less, 0.7 wt.% or less, 0.6 wt.% or less, 0.5 wt.% or less, 0.4 wt.% or less, 0.3 wt.% or less, 0.2 wt.% or less, 0.1 wt.% or less, 0.05 wt.% or less, 0.02 wt.% or less, or 0.01 wt.% or less. In some embodiments, the absolute value of the difference between a first concentration of available iodine of the initial iodophor composition and a second concentration of available iodine of the sterilized iodophor composition is in a range from 0.01 wt.% to 0.05 wt.%, 0.01 wt.% to 0.1 wt.%, 0.01 wt.% to 0.2 wt.%, 0.01 wt.% to 0.3 wt.%, 0.01 wt.% to 0.4 wt.%, 0.01 wt.% to 0.5 wt.%, 0.01 wt.% to 0.6 wt.%, 0.01 wt.% to 0.7 wt.%, 0.01 wt.% to 0.8 wt.%, 0.01 wt.% to 0.9 wt.%, 0.01 wt.% to 1 wt.%, 0.05 wt.% to 0.1 wt.%, 0.05 wt.% to 0.5 wt.%, 0.05 wt.% to 1 wt.%, 0.1 wt.% to 0.5 wt.%, 0.1 wt.% to 1 wt.%, or 0.5 wt.% to 1 wt.%.

In some embodiments, the sterilized iodophor composition comprises water and has an acidic or neutral pH. In some embodiments, the sterilized iodophor composition has a pH of at least 1.5, at least 2.0, at least 2.5, at least 3.0, at least 3.5, at least 4.0, at least 4.5, at least 5.0, at least 5.5, at least 6.0, at least 6.5, or at least 7.0. In some embodiments, the sterilized iodophor composition has a pH of 7.0 or less, 6.5 or less, 6.0 or less, 5.5 or less, 5.0 or less, 4.5 or less, 4.0 or less, 3.5 or less, 3.0 or less, 2.5 or less, 2.0 or less, or 1.5 or less. In some embodiments, the sterilized iodophor composition has a pH in a range from 1.5 to 3.0, 1.5 to 4.0, 1.5 to 5.0, 1.5 to 6.0, 1.5 to 6.5, 1.5 to 7.0, 2.0 to 4.0, 2.0 to 5.0, 2.0 to 5.5, 2.0 to 6.0, 2.0 to 6.5, 2.0 to 7.0, 3.0 to 5.0, 3.0 to 6.0, 3.0 to 6.5, 3.0 to 7.0, 4.0 to 6.0, 4.0 to 6.5, 4.0 to 7.0, 5.0 to 6.0, 5.0 to 6.5, 5.0 to 7.0, or 6.0 to 7.0.

In some embodiments, any difference between the pH values of the initial iodophor composition and the sterilized iodophor composition is relatively small. In some cases, minimizing any change in pH caused by sterilization may be advantageous because the pH of an iodophor composition may significantly impact the potency of the composition, patient comfort, and/or patient safety. As an illustrative example, a highly acidic or basic iodophor composition may cause irritation when applied to a patient's skin. In some embodiments, the absolute value of the difference between the pH of the initial iodophor composition and the pH of the sterilized iodophor composition is 0.5 or less, 0.4 or less, 0.3 or less, 0.2 or less, 0.1 or less, 0.05 or less, 0.02 or less, 0.01 or less, or about 0.0. In some embodiments, the absolute value of the difference between the pH of the initial iodophor composition and the pH of the sterilized iodophor composition is in a range from about 0.0 to 0.01, about 0.0 to 0.02, about 0.0 to 0.05, about 0.0 to 0.1, about 0.0 to 0.2, about 0.0 to 0.3, about 0.0 to 0.4, about 0.0 to 0.5, about 0.01 to 0.02, about 0.01 to 0.05, about 0.01 to 0.1, about 0.01 to 0.2, about 0.01 to 0.3, about 0.01 to 0.4, about 0.01 to 0.5, about 0.05 to 0.1, about 0.05 to 0.2, about 0.05 to 0.3, about 0.05 to 0.4, about 0.05 to 0.5, about 0.1 to 0.2, about 0.1 to 0.3, about 0.1 to 0.4, about 0.1 to 0.5, or about 0.2 to 0.5.

In some embodiments, the sterilized iodophor composition has a viscosity at ambient temperature (i.e., about 20°C) that facilitates application of the sterilized iodophor composition to one or more locations on a patient. In some embodiments, the sterilized iodophor composition has a viscosity at ambient temperature (i.e., about 20°C) of at least 500 centipoise (cP), at least 600 cP, at least 700 cP, at least 800 cP, at least 900 cP, at least 1000 cP, at least 1500 cP, at least 2000 cP, at least 2500 cP, or at least 3000 cP. In some embodiments, the sterilized iodophor composition has a viscosity at ambient temperature of 3000 cP or less, 2500 cP or less, 2000 cP or less, 1500 cP or less, 1000 cP or less, or 500 cP or less. In some embodiments, the sterilized iodophor composition has a viscosity at ambient temperature in a range from 500 cP to 1000 cP, 500 cP to 1500 cP, 500 cP to 2000 cP, 500 cP to 2500 cP, 500 cP to 3000 cP, 1000 cP to 1500 cP, 1000 cP to 2000 cP, 1000 cP to 2500 cP, 1000 cP to 3000 cP, 1500 cP to 2000 cP, 1500 cP to 2500 cP, 1500 cP to 3000 cP, 2000 cP to 2500 cP, or 2000 cP to 3000 cP.

In some embodiments, any difference between the viscosity of the initial iodophor composition at ambient temperature and the viscosity of the sterilized iodophor composition at ambient temperature is relatively small. In some cases, minimizing any change in viscosity caused by sterilization may be advantageous because the viscosity of an iodophor composition may significantly impact application of the iodophor composition to a patient. For example, the viscosity of an iodophor composition may prevent pooling and/or provide improved control over application of the composition. In some embodiments, the percent difference between the viscosity of the initial iodophor composition and the viscosity of the sterilized iodophor composition is 60% or less, 50% or less, 40% or less, 30% or less, 20% or less, 10% or less, 5% or less, 2% or less, 1% or less, or about 0%. In some embodiments, the percent difference between the viscosity of the initial iodophor composition and the viscosity of the sterilized iodophor composition is in a range from 0% to 1%, 0% to 2%, 0% to 5%, 0% to 10%, 0% to 20%, 0% to 30%, 0% to 40%, 0% to 50%, 0% to 60%, 1% to 2%, 1% to 5%, 1% to 10%, 1% to 20%, 1% to 30%, 1% to 40%, 1% to 50%, 1% to 60%, 5% to 10%, 5% to 20%, 5% to 30%, 5% to 40%, 5% to 50%, 5% to 60%, 10% to 20%, 10% to 30%, 10% to 40%, 10% to 50%, 10% to 60%, 20% to 30%, 20% to 40%, 20% to 50%, 20% to 60%, 30% to 40%, 30% to 50%, 30% to 60%, 40% to 50%, 40% to 60%, or 50% to 60%.

In certain embodiments, the initial and/or sterilized iodophor composition comprises water. In some instances, the water is deionized water and/or distilled water. In some embodiments, the initial and/or sterilized iodophor composition comprises water in a concentration of at least 5 wt.%, at least 10 wt.%, at least 15 wt.%, at least 20 wt.%, at least 30 wt.%, at least 40 wt.%, at least 50 wt.%, at least 60 wt.%, at least 70 wt.%, at least 80 wt.%, at least 90 wt.%, or at least 95 wt.%. In some embodiments, the initial and/or sterilized iodophor composition comprises water in a concentration in a range from 5 wt.% to 20 wt.%, 5 wt.% to 30 wt.%, 5 wt.% to 40 wt.%, 5 wt.% to 50 wt.%, 5 wt.% to 60 wt.%, 5 wt.% to 70 wt.%, 5 wt.% to 80 wt.%, 5 wt.% to 90 wt.%, 5 wt.% to 95 wt.%, 10 wt.% to 20 wt.%, 10 wt.% to 30 wt.%, 10 wt.% to 40 wt.%, 10 wt.% to 50 wt.%, 10 wt.% to 60 wt.%, 10 wt.% to 70 wt.%, 10 wt.% to 80 wt.%, 10 wt.% to 90 wt.%, 10 wt.% to 95 wt.%, 20 wt.% to 40 wt.%, 20 wt.% to 50 wt.%, 20 wt.% to 60 wt.%, 20 wt.% to 70 wt.%, 20 wt.% to 80 wt.%, 20 wt.% to 90 wt.%, 20 wt.% to 95 wt.%, 30 wt.% to 50 wt.%, 30 wt.% to 60 wt.%, 30 wt.% to 70 wt.%, 30 wt.% to 80 wt.%, 30 wt.% to 90 wt.%, 30 wt.% to 95 wt.%, 40 wt.% to 60 wt.%, 40 wt.% to 70 wt.%, 40 wt.% to 80 wt.%, 40 wt.% to 90 wt.%, 40 wt.% to 95 wt.%, 50 wt.% to 70 wt.%, 50 wt.% to 80 wt.%, 50 wt.% to 90 wt.%, 50 wt.% to 95 wt.%, 60 wt.% to 80 wt.%, 60 wt.% to 90 wt.%, 60 wt.% to 95 wt.%, 70 wt.% to 90 wt.%, 70 wt.% to 95 wt.%, 80 wt.% to 90 wt.%, or 80 wt.% to 95 wt.%.

In some embodiments, the initial and/or sterilized iodophor composition comprises an alcohol. Non-limiting examples of suitable alcohols include methanol, ethanol, n-propanol, isopropanol, n-butanol, isobutanol, sec-butanol, tert-butanol, benzyl alcohol, phenol, and combinations thereof. In certain embodiments, the alcohol comprises ethanol, isopropanol, and/or n-propanol. In some cases, it may be advantageous for the initial and/or sterilized iodophor composition to comprise an alcohol because the alcohol can act as an antimicrobial agent. In some embodiments, the initial and/or sterilized iodophor composition comprises an alcohol in a concentration of at least 5 wt.%, at least 10 wt.%, at least 15 wt.%, at least 20 wt.%, at least 30 wt.%, at least 40 wt.%, at least 50 wt.%, at least 60 wt.%, at least 70 wt.%, at least 80 wt.%, at least 90 wt.%, or at least 95 wt.%. In some embodiments, the initial and/or sterilized iodophor composition comprises an alcohol in a concentration in a range from 5 wt.% to 20 wt.%, 5 wt.% to 30 wt.%, 5 wt.% to 40 wt.%, 5 wt.% to 50 wt.%, 5 wt.% to 60 wt.%, 5 wt.% to 70 wt.%, 5 wt.% to 80 wt.%, 5 wt.% to 90 wt.%, 5 wt.% to 95 wt.%, 10 wt.% to 20 wt.%, 10 wt.% to 30 wt.%, 10 wt.% to 40 wt.%, 10 wt.% to 50 wt.%, 10 wt.% to 60 wt.%, 10 wt.% to 70 wt.%, 10 wt.% to 80 wt.%, 10 wt.% to 90 wt.%, 10 wt.% to 95 wt.%, 20 wt.% to 40 wt.%, 20 wt.% to 50 wt.%, 20 wt.% to 60 wt.%, 20 wt.% to 70 wt.%, 20 wt.% to 80 wt.%, 20 wt.% to 90 wt.%, 20 wt.% to 95 wt.%, 30 wt.% to 50 wt.%, 30 wt.% to 60 wt.%, 30 wt.% to 70 wt.%, 30 wt.% to 80 wt.%, 30 wt.% to 90 wt.%, 30 wt.% to 95 wt.%, 40 wt.% to 60 wt.%, 40 wt.% to 70 wt.%, 40 wt.% to 80 wt.%, 40 wt.% to 90 wt.%, 40 wt.% to 95 wt.%, 50 wt.% to 70 wt.%, 50 wt.% to 80 wt.%, 50 wt.% to 90 wt.%, 50 wt.% to 95 wt.%, 60 wt.% to 80 wt.%, 60 wt.% to 90 wt.%, 60 wt.% to 95 wt.%, 70 wt.% to 90 wt.%, 70 wt.% to 95 wt.%, 80 wt.% to 90 wt.%, or 80 wt.% to 95 wt.%.

In some embodiments, the initial and/or sterilized iodophor composition comprises one or more polymer-forming agents. In certain embodiments, a polymer-forming agent comprises a copolymer. Examples of a suitable polymer-forming agent include, but are not limited to, a methacrylic acid-methyl methacrylate copolymer, a carboxylated acrylic copolymer, and hydroxypropylcellulose. In some embodiments, the initial and/or sterilized iodophor composition comprises a polymer-forming agent in a concentration of at least 0.1 wt.%, at least 0.2 wt.%, at least 0.5 wt.%, at least 1 wt.%, at least 1.5 wt.%, at least 2 wt.%, at least 5 wt.%, or at least 10 wt.%. In some embodiments, the initial and/or sterilized iodophor composition comprises a polymer-forming agent in a concentration in a range from 0.1 wt.% to 0.5 wt.%, 0.1 wt.% to 1 wt.%, 0.1 wt.% to 2 wt.%, 0.1 wt.% to 5 wt.%, 0.1 wt.% to 10 wt.%, 0.2 wt.% to 1 wt.%, 0.2 wt.% to 2 wt.%, 0.2 wt.% to 5 wt.%, 0.2 wt.% to 10 wt.%, 0.5 wt.% to 1 wt.%, 0.5 wt.% to 2 wt.%, 0.5 wt.% to 5 wt.%, 0.5 wt.% to 10 wt.%, 1 wt.% to 2 wt.%, 1 wt.% to 5 wt.%, 1 wt.% to 10 wt.%, 2 wt.% to 5 wt.%, 2 wt.% to 10 wt.%, or 5 wt.% to 10 wt.%.

In some embodiments, the initial and/or sterilized iodophor composition comprises a total concentration of polymer-forming agents of at least 0.1 wt.%, at least 0.2 wt.%, at least 0.5 wt.%, at least 1 wt.%, at least 1.5 wt.%, at least 2 wt.%, at least 5 wt.%, or at least 10 wt.%. In some embodiments, the initial and/or sterilized iodophor composition comprises a total concentration of polymer-forming agents in a range from 0.1 wt.% to 0.5 wt.%, 0.1 wt.% to 1 wt.%, 0.1 wt.% to 2 wt.%, 0.1 wt.% to 5 wt.%, 0.1 wt.% to 10 wt.%, 0.2 wt.% to 1 wt.%, 0.2 wt.% to 2 wt.%, 0.2 wt.% to 5 wt.%, 0.2 wt.% to 10 wt.%, 0.5 wt.% to 1 wt.%, 0.5 wt.% to 2 wt.%, 0.5 wt.% to 5 wt.%, 0.5 wt.% to 10 wt.%, 1 wt.% to 2 wt.%, 1 wt.% to 5 wt.%, 1 wt.% to 10 wt.%, 2 wt.% to 5 wt.%, 2 wt.% to 10 wt.%, or 5 wt.% to 10 wt.%.

In some embodiments, the initial and/or sterilized iodophor composition comprises one or more pH-adjusting agents. The pH-adjusting agent may be an acid, a base, or a buffer salt. Non-limiting examples of suitable acidic pH-adjusting agents include citric acid, lactic acid, and acetic acid. Non-limiting examples of suitable basic pH-adjusting agents include 2-amino-2-methyl-1-propanol (e.g., AMP-95), sodium hydroxide, potassium hydroxide, and disodium phosphate. In some embodiments, the initial and/or sterilized iodophor composition comprises a pH-adjusting agent in a concentration of at least 0.01 wt.%, at least 0.02 wt.%, at least 0.05 wt.%, at least 0.1 wt.%, at least 0.2 wt.%, at least 0.5 wt.%, at least 1 wt.%, at least 2 wt.%, or at least 5 wt.%. In some embodiments, the initial and/or sterilized iodophor composition comprises a pH-adjusting agent in a concentration in a range from 0.01 wt.% to 0.05 wt.%, 0.01 wt.% to 0.1 wt.%, 0.01 wt.% to 0.5 wt.%, 0.01 wt.% to 1 wt.%, 0.01 wt.% to 2 wt.%, 0.01 wt.% to 5 wt.%, 0.05 wt.% to 0.1 wt.%, 0.05 wt.% to 0.5 wt.%, 0.05 wt.% to 1 wt.%, 0.05 wt.% to 2 wt.%, 0.05 wt.% to 5 wt.%, 0.1 wt.% to 0.5 wt.%, 0.1 wt.% to 1 wt.%, 0.1 wt.% to 2 wt.%, 0.1 wt.% to 5 wt.%, 0.5 wt.% to 1 wt.%, 0.5 wt.% to 2 wt.%, 0.5 wt.% to 5 wt.%, or 1 wt.% to 5 wt.%.

In some embodiments, the initial and/or sterilized iodophor composition comprises one or more additional components. In certain embodiments, the initial and/or sterilized iodophor composition comprises one or more surfactants. A non-limiting example of a suitable surfactant is nonoxynol-9. In certain embodiments, the initial and/or sterilized iodophor composition comprises one or more thickening and/or gelling agents. Non-limiting examples of suitable thickening and/or gelling agents include hydroxypropyl cellulose, simethicone, sodium carboxymethyl cellulose, and polyethylene glycol ("PEG"). In certain embodiments, the initial and/or sterilized iodophor composition comprises one or more irritation-reducing agents. Non-limiting examples of suitable irritation-reducing agents include glycerin, petroleum jelly, mineral oil, and glycerol. In some embodiments, the initial and/or sterilized iodophor composition further comprises one or more dyes and/or emulsifiers. In some embodiments, the iodophor composition comprises one or more components that advantageously enhance polymer stability. In certain instances, one or more components of the iodophor composition may serve multiple functions (e.g., a single component may both reduce irritation and enhance polymer stability).

Iodophor compositions described herein may be administered in any suitable amount. In some embodiments, an amount of an iodophor composition administered to a patient is at least 0.01 mL, at least 0.05 mL, at least 0.1 mL, at least 0.5 mL, at least 0.67 mL, at least 0.75 mL, at least 1 mL, at least 1.5 mL, at least 3 mL, at least 5 mL, at least 10 mL, at least 10.5 mL, at least 13 mL, at least 26 mL, at least 30 mL, at least 60 mL, or at least 100 mL. In some embodiments, an amount of an iodophor composition administered to a patient is in a range from 0.01 mL to 0.1 mL, 0.01 mL to 0.5 mL, 0.01 mL to 1 mL, 0.01 mL to 3 mL, 0.01 mL to 5 mL, 0.01 mL to 10 mL, 0.01 to 13 mL, 0.01 to 30 mL, 0.01 mL to 60 mL, 0.01 mL to 100 mL, 0.1 mL to 1 mL, 0.1 mL to 3 mL, 0.1 mL to 5 mL, 0.1 mL to 10 mL, 0.1 mL to 13 mL, 0.1 mL to 30 mL, 0.1 mL to 60 mL, 0.1 mL to 100 mL, 1 mL to 5 mL, 1 mL to 10 mL, 1 mL to 13 mL, 1 mL to 30 mL, 1 mL to 60 mL, 1 mL to 100 mL, 5 mL to 10 mL, 5 mL to 13 mL, 5 mL to 30 mL, 5 mL to 60 mL, 5 mL to 100 mL, 10 mL to 30 mL, 10 mL to 60 mL, 10 mL to 100 mL, 30 mL to 60 mL, 30 mL to 100 mL, or 60 mL to 100 mL.

Iodophor compositions described herein may be administered using any applicator known in the art. Examples of suitable applicators are described in U.S. Patent No. 7,993,066, by Flores et al., filed May 18, 2010, entitled "Liquid Applicator and Method for Reducing the Concentration of By-Products from Antiseptic"; and U.S. Patent No. 9,757,551, by Degala et al., filed Oct. 4, 2013, entitled "Antiseptic Applicator," the contents of which are incorporated herein by reference in their entireties for all purposes.

### Example 1

In this Example, the D-values (i.e., time required to reduce the challenge organism, *Geobacillus stearothermophilus,* counts by one log) at three temperatures (105°C, 110°C, and 115°C) were determined for a Prevail-FX® formulation comprising 8.3% povidone-iodine in 72.5% isopropanol (i.e., an iodophor composition comprising PVP-I) and compared against the D-values for: (1) a solution of 70% isopropanol; and (2) a ChloraPrep™ formulation comprising 2% chlorhexidine gluconate in 70% isopropanol (i.e., an antiseptic formulation comprising two active ingredients).

**Table 1**

| | **Prevail- FX®** | **70% Isopropanol** | **ChloraPrep™** |
|---|---|---|---|
| Temperature | D Value (minutes) | D Value (minutes) | D Value (minutes) |
| 105°C | 0.4476 | 4.8100 | 1.29 |
| 110°C | 0.2501 | 1.3738 | 0.5 |
| 115°C | 0.2349 | 0.4626 | 0.243 |

The results shown in Table 1 demonstrate that the D-values for the 70% isopropanol solution were twice as high as those found for the Prevail-FX® formulation at all three temperatures investigated, over five times higher at 110°C, and over ten times higher at 105°C. These results demonstrate that the unique sterilization range of the alcoholic PVP-I solution of Prevail-FX® cannot be explained by a 70% isopropanol solution.

The results shown in Table 1 also demonstrate that the D-values for the sterilization of ChloraPrep™ were significantly higher than for the alcoholic PVP-I solution of Prevail-FX®. Like Prevail-FX®, ChloraPrep™ contains two active antimicrobial agents: chlorhexidine gluconate and isopropanol. These results demonstrate that the unique sterilization range of the Prevail-FX® formulation cannot be explained by the presence of two active ingredients. The milder sterilization conditions offer a unique opportunity to preserve potency and other key formulation attributes.

### Example 2

In this Example, an oil bath was used to simulate the efficient heat transfer characteristic of a moist heat sterilization process (i.e., cascading water sterilization process). Samples of six test formulations were hermetically sealed in glass ampoules. Each glass ampoule, which had a maximum capacity of approximately 10 mL, contained approximately 5 to 8 mL of one of the six test formulations. Each glass ampoule was formed from USP Type I borosilicate glass and had a diameter of 19 mm. The glass ampoules were then submerged in a recirculating oil bath pre-equilibrated to a set temperature for a specified amount of time and were quickly removed and quenched in a cold water bath prior to chemical analysis. It was estimated that the temperatures of the formulations within the glass ampoules reached thermal equilibrium with the recirculating oil in less than about 30 seconds.

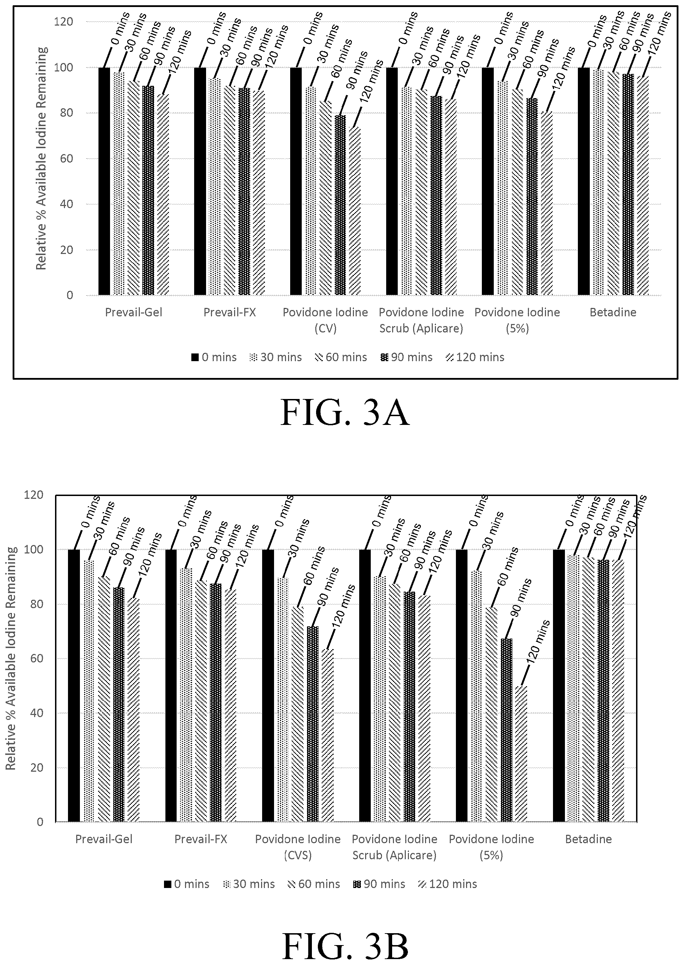

Six formulations comprising povidone-iodine were exposed to 110°C and 115°C regimens as a function of time, and the percentage of available iodine remaining in solution was measured by titrimetry using sodium thiosulfate. Results at 110°C are shown in Table 2 and FIG. 3A, and results at 115°C are shown in Table 3 and FIG. 3B.

| Table 2: Percentage of Available Iodine in PVP-I as a Function of Time at 110°C | | | | | |
|---|---|---|---|---|---|
| **Formulation** | Time (mins) | | | | |
| | 0 | 30 | 60 | 90 | 120 |
| | % Available Iodine | | | | |
| Prevail™ (gel) | 0.51 | 0.50 | 0.48 | 0.47 | 0.45 |
| Prevail-FX® | 0.89 | 0.85 | 0.82 | 0.81 | 0.8 |
| Povidone- iodine (Walgreens) | 0.96 | 0.88 | 0.82 | 0.76 | 0.71 |
| Povidone- iodine scrub (Aplicare) | 0.72 | 0.66 | 0.65 | 0.63 | 0.62 |
| Povidone- iodine 5% (Aplicare) | 0.52 | 0.49 | 0.47 | 0.45 | 0.42 |
| Betadine® | 1.09 | 1.08 | 1.07 | 1.06 | 1.05 |

| Table 3: Percentage of Available Iodine in PVP-I as a Function of Time at 115°C | | | | | |
|---|---|---|---|---|---|
| **Formulation** | Time (mins) | | | | |
| | 0 | 30 | 60 | 90 | 120 |
| | % Available Iodine | | | | |
| Prevail™ (gel) | 0.51 | 0.49 | 0.46 | 0.44 | 0.42 |
| Prevail-FX® | 0.89 | 0.83 | 0.79 | 0.78 | 0.76 |
| Povidone- iodine | 0.96 | 0.86 | 0.76 | 0.69 | 0.61 |
| (Walgreens) | | | | | |
| Povidone- iodine scrub (Aplicare) | 0.72 | 0.65 | 0.63 | 0.61 | 0.60 |
| Povidone- iodine 5% (Aplicare) | 0.52 | 0.48 | 0.41 | 0.35 | 0.26 |
| Betadine® | 1.09 | 1.07 | 1.06 | 1.05 | 1.05 |

Tables 2 and 3, along with FIGS. 3A and 3B, demonstrate the reduction in percentage of available iodine as a function of time at 110°C and 115°C.

Additionally, the percentage of available iodine for the same six formulations was compared for 3 minutes at 110°C and 15 minutes at 121°C. These results are shown in Table 4.

| Table 4: Percentage of Available Iodine in PVP-I-containing formulations at 110°C for 3 minutes and 121°C for 15 minutes | | | |
|---|---|---|---|
| Formulation | Time (mins) | | |
| | 0 | 3 minutes at 110°C | 15 minutes at 121°C |
| | % Available Iodine | | |
| Prevail™ (gel) | 0.51 | 0.51 | 0.49 |
| Prevail-FX® | 0.86 | 0.85 | 0.82 |
| Povidone- iodine (Walgreens) | 1.03 | 1.03 | 0.97 |
| Povidone- iodine scrub (Aplicare) | 0.69 | 0.69 | 0.64 |
| Povidone- iodine 5% (Aplicare) | 0.88 | 0.88 | 0.82 |
| Betadine® | 0.50 | 0.50 | 0.46 |

These results demonstrate that heating iodophor compositions for 3 minutes at 110°C results in higher percentages of available iodine than heating the same compositions for 15 minutes at 121°C.

### Example 3

In this Example, the pH values of various formulations before and after heat treatment were investigated. This Example demonstrates that heating PVP-I formulations at 110°C for either 3 minutes or 12 minutes generally has less of an impact on pH than heating the same formulations at 121°C for 15 minutes. This Example also demonstrates that heating PVP-I formulations at 110°C for 3 minutes generally results in a smaller change in pH than heating the same formulations at 110°C for 12 minutes.

The results are shown in Tables 5 and 6.

| Table 5: pH Values Following Heating at 110°C for 12 Minutes | | | |
|---|---|---|---|
| Formulation | Untreated | Heat- Treated | Δ pH |
| Aplicare PVP-I Scrub | 4.68 | 4.73 | -0.04 |
| Aplicare 10% PVP-I Solution | 5.32 | 5.07 | 0.26 |
| PVP-I (Walgreens) | 4.38 | 3.94 | 0.44 |
| Prevail™ (gel) | 3.61 | 3.52 | 0.09 |
| Prevail-FX® | 2.70 | 2.55 | 0.15 |
| Betadine® | 3.47 | 3.29 | 0.19 |

| Table 6: pH of PVP-1-containing formulations at 110°C for 3 minutes and 121°C for 15 minutes | | | |
|---|---|---|---|
| Formulation | Time (mins) | | |
| | 0 | 3 minutes at 110°C | 15 minutes at 121°C |
| | pH | | |
| Prevail™ (gel) | 3.651 | 3.673 | 3.420 |
| Prevail-FX® | 2.937 | 2.854 | 2.735 |
| Povidone- iodine (Walgreens) | 3.350 | 3.351 | 3.032 |
| Povidone- iodine scrub (Aplicare) | 4.550 | 4.490 | 4.125 |
| Povidone- iodine 5% (Aplicare) | 4.741 | 4.728 | 4.420 |
| Betadine® | 4.634 | 4.595 | 3.805 |
| DuraPrep™ | 4.754 | 4.505 | 3.455 |

### Comparative Example 1

In this Comparative Example, the viscosity of Prevail™ gel, which comprises 5% povidone-iodine in 62% ethanol, before and after heating at 121°C for 20 minutes was investigated. The results are shown in Table 7. These results demonstrate that solution sterilization at these conditions significantly reduced the viscosity of the product.

| Table 7: Viscosity Following Heating at 121°C for 20 Minutes | | | |
|---|---|---|---|
| Formulation | Viscosity (cP) (before) | Viscosity (cP) (after) | % Reduction |
| Prevail™ (gel) | 2190 | 579 | 73% |

### Comparative Example 2

In this Comparative Example, the appearance of an iodophor composition after heat treatment was investigated. A DuraPrep™ formulation manufactured by 3M was heated at 121°C for 12 minutes. This heating resulted in formation of a precipitate. One potential cause for the formation of the precipitate is the loss of solubility of the polymeric excipient. The formation of this precipitate is an undesirable consequence of solution sterilization and would be significantly reduced or eliminated through the use of less thermally stressful sterilization conditions at 110°C or 115°C.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present invention.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

### EMBODIMENTS

The present invention relates to the following embodiments:
1. A sterilized iodophor composition, wherein the sterilized iodophor composition is sterilized by a method comprising:
   heating an initial iodophor composition to produce the sterilized iodophor composition, wherein the sterilized iodophor composition has a sterility assurance level of 10⁻³ or less.
2. A sterilized iodophor composition, comprising:
   at least 5.0% by weight of an iodine-polymer complex,
   wherein the sterilized iodophor composition has a sterility assurance level of 10⁻³ or less and a viscosity of 3000 cP or less.
3. A sterilization method, comprising:
   heating an initial iodophor composition to produce a sterilized iodophor composition having a sterility assurance level of 10⁻³ or less.
4. The composition or method of any preceding embodiment, wherein the initial iodophor composition comprises an iodine-polymer complex.
5. The composition or method of embodiment 4, wherein the iodine-polymer complex is PVP-I or iodine povacrylex.
6. The composition or method of embodiment 5, wherein the iodine-polymer complex is PVP-I.
7. The composition or method of any preceding embodiment, wherein the sterilized iodophor composition comprises an iodine-polymer complex.
8. The composition or method of embodiment 7, wherein the iodine-polymer complex is PVP-I or iodine povacrylex.
9. The composition or method of embodiment 8, wherein the iodine-polymer complex is PVP-I.
10. The composition or method of any preceding embodiment, wherein the sterilized composition has a sterility assurance level of 10⁻⁶ or less.
11. The composition or method of any preceding embodiment, wherein the sterilized iodophor composition comprises water.
12. The composition or method of embodiment 11, wherein the sterilized iodophor composition comprises water in a concentration of at least 10 wt.%.
13. The composition or method of embodiment 12, wherein the sterilized iodophor composition comprises water in a concentration of at least 50 wt.%.
14. The composition or method of embodiment 13, wherein the sterilized iodophor composition comprises water in a concentration of at least 70 wt.%.
15. The composition or method of any preceding embodiment, wherein the sterilized iodophor composition comprises an alcohol.
16. The composition or method of embodiment 15, wherein the alcohol is isopropanol or ethanol.
17. The composition or method of any preceding embodiment, wherein heating the initial iodophor composition comprises exposing the initial iodophor composition to a sterilization temperature in a range from 65°C to 121°C for a sterilization time period in a range from 1.5 minutes to 15 minutes.
18. The composition or method of any preceding embodiment, wherein the sterilization temperature is in a range from 85°C to 115°C.
19. The composition or method of embodiment 18, wherein the sterilization temperature is in a range from 110°C to 115°C.
20. The composition or method of any preceding embodiment, wherein the sterilization time period is in a range from 1.5 minutes to 10 minutes.
21. The composition or method of embodiment 20, wherein the sterilization time period is in a range from 1.5 minutes to 6 minutes.
22. The composition or method of embodiment 21, wherein the sterilization time period is in a range from 1.5 minutes to 3 minutes.
23. The composition or method of any preceding embodiment, wherein the initial iodophor composition has a first concentration of available iodine and the sterilized iodophor composition has a second concentration of available iodine, and wherein the percent difference between the first concentration of available iodine and the second concentration of available iodine is 8% or less.
24. The composition or method of embodiment 23, wherein the percent difference between the first concentration of available iodine and the second concentration of available iodine is 5% or less.
25. The composition or method of any one of embodiments 23-24, wherein the absolute value of the difference between the first concentration of available iodine and the second concentration of available iodine is 0.1 wt.% or less.
26. The composition or method of any one of embodiments 23-25, wherein the second concentration of available iodine is at least 0.5%.
27. The composition or method of any preceding embodiment, wherein the initial iodophor composition has a first pH and the sterilized iodophor composition has a second pH, and wherein the absolute value of the difference between the first pH and the second pH is 0.5 or less.
28. The composition or method of embodiment 27, wherein the absolute value of the difference between the first pH and the second pH is 0.2 or less.
29. The composition or method of any one of embodiments 27-28, wherein the second pH is in a range from 1.5 to 6.5.
30. The composition or method of embodiment 29, wherein the second pH is in a range from 2.0 to 5.5.
31. The composition or method of any preceding embodiment, wherein the initial iodophor composition has a first viscosity and the sterilized iodophor composition has a second viscosity, and wherein the percent difference between the first viscosity and the second viscosity is 50% or less.
32. The composition or method of any preceding embodiment, wherein the method further comprises providing a container containing the initial iodophor composition.
33. The composition or method of any preceding embodiment, wherein exposing the initial composition to the sterilization temperature for the sterilization time period comprises exposing at least a portion of a container containing the initial iodophor composition to a heated liquid.
34. The composition or method of embodiment 33, wherein the heated liquid comprises water.
35. The composition or method of any preceding embodiment, wherein the method further comprises heating the initial iodophor composition using a cascading water sterilizer.
36. The composition or method of any preceding embodiment, wherein exposing the initial iodophor composition to the sterilization temperature for the sterilization time period comprises contacting at least a portion of a container containing the initial iodophor composition with a cascading waterfall stream produced by a cascading water sterilizer for the duration of the sterilization time period.
37. The composition or method of any preceding embodiment, wherein the method further comprises terminating exposure to the sterilization temperature upon expiration of the sterilization time period.
38. The composition or method of any one of embodiments 32-37, wherein the container is sealed during the heating step.
39. The composition or method of any one of embodiments 32-38, wherein the container comprises glass.
40. The composition or method of any one of embodiments 32-39, wherein the container is a pouch.
41. The composition or method of embodiment 40, wherein the pouch comprises a polymer.
42. The composition or method of any one of embodiments 40-41, wherein the pouch comprises a metallic laminate.
43. The composition or method of any preceding embodiment, wherein the sterilized iodophor composition comprises an alcohol in a concentration of at least 50 wt.%.
44. The composition or method of embodiment 43, wherein the sterilized iodophor composition comprises an alcohol in a concentration of at least 70 wt.%.
45. The composition or method of any preceding embodiment, wherein the sterilized iodophor composition comprises PVP-I in a concentration of at least 5 wt.%.
46. The composition or method of embodiment 45, wherein the sterilized iodophor composition comprises PVP-I in a concentration of at least 7 wt.%.
47. The composition or method of any preceding embodiment, wherein the sterilized iodophor composition comprises iodine povacrylex in a concentration of at least 5 wt.%.
48. The composition or method of embodiment 47, wherein the sterilized iodophor composition comprises iodine povacrylex in a concentration of at least 7 wt.%.
49. The composition or method of any preceding embodiment, wherein the sterilized iodophor composition comprises a concentration of available iodine of at least 0.5 wt.%.
50. The composition or method of any preceding embodiment, wherein the sterilized iodophor composition comprises a concentration of available iodine in a range from 0.5 wt.% to 1 wt.%.
51. The composition or method of any preceding embodiment, wherein the sterilized iodophor composition comprises one or more surfactants, one or more polymer-forming agents, and/or one or more pH-adjusting agents.

## Claims

1. A sterilized iodophor composition, wherein the sterilized iodophor composition is sterilized by a method comprising:
heating an initial iodophor composition to produce the sterilized iodophor composition, wherein the sterilized iodophor composition has a sterility assurance level of 10⁻³ or less, optionally wherein the sterilized iodophor composition has a sterility assurance level of 10⁻⁶ or less.

2. The composition of claim 1, wherein heating the initial iodophor composition comprises exposing the initial iodophor composition to a sterilization temperature in a range from 65°C to 121°C for a sterilization time period in a range from 1.5 minutes to 15 minutes.

3. The composition of claim 2, wherein the sterilization temperature is in a range from 85°C to 115°C, optionally wherein the sterilization temperature is in a range from 110°C to 115°C.

4. The composition of any one of claims 2-3, wherein the sterilization time period is in a range from 1.5 minutes to 10 minutes, optionally wherein the sterilization time period is in a range from 1.5 minutes to 6 minutes, optionally wherein the sterilization time period is in a range from 1.5 minutes to 3 minutes.

5. The composition of any one of claims 2-4, wherein exposing the initial iodophor composition to the sterilization temperature for the sterilization time period comprises exposing at least a portion of a container containing the initial iodophor composition to a heated liquid, optionally wherein exposing the initial iodophor composition to the sterilization temperature for the sterilization time period comprises contacting at least a portion of the container containing the initial iodophor composition with a cascading waterfall stream produced by a cascading water sterilizer for the duration of the sterilization time period.

6. The composition of any one of claims 1-5, wherein the initial iodophor composition has a first concentration of available iodine and the sterilized iodophor composition has a second concentration of available iodine, and wherein the percent difference between the first concentration of available iodine and the second concentration of available iodine is 8% or less, optionally wherein the percent difference between the first concentration of available iodine and the second concentration of available iodine is 5% or less, optionally wherein the absolute value of the difference between the first concentration of available iodine and the second concentration of available iodine is 0.1 wt% or less.

7. The composition of any one of claims 1-6, wherein the initial iodophor composition has a first pH and the sterilized iodophor composition has a second pH, and wherein the absolute value of the difference between the first pH and the second pH is 0.5 or less, optionally wherein the absolute value of the difference between the first pH and the second pH is 0.2 or less.

8. A sterilized iodophor composition, comprising:
at least 5.0% by weight of an iodine-polymer complex,
wherein the sterilized iodophor composition has a sterility assurance level of 10⁻³ or less and a viscosity of 3000 cP or less.

9. The composition of any one of claims 1-8, wherein the sterilized iodophor composition comprises an iodine-polymer complex, optionally wherein the iodine-polymer complex is PVP-I or iodine povacrylex.

10. The composition of any one of claims 1-9, wherein the sterilized iodophor composition comprises PVP-I or iodine povacrylex in a concentration of at least 5 wt%, optionally wherein the sterilized iodophor composition comprises PVP-I or iodine povacrylex in a concentration of at least 7 wt%.

11. The composition of any one of claims 1-10, wherein the sterilized iodophor composition comprises a concentration of available iodine of at least 0.5 wt%, optionally wherein the sterilized iodophor composition comprises a concentration of available iodine in a range from 0.5 wt% to 1 wt%.

12. The composition of any one of claims 1-11, wherein the sterilized iodophor composition has a pH in a range from 1.5 to 6.5, optionally wherein the sterilized iodophor composition has a pH in a range from 2.0 to 5.5.

13. A sterilization method, comprising:
heating an initial iodophor composition to produce a sterilized iodophor composition having a sterility assurance level of 10⁻³ or less, optionally wherein the sterilized composition has a sterility assurance level of 10⁻⁶ or less.

14. The method of claim 13, wherein heating the initial iodophor composition comprises exposing the initial iodophor composition to a sterilization temperature for a sterilization time period, optionally wherein exposing the initial iodophor composition to the sterilization temperature for the sterilization time period comprises contacting at least a portion of a container containing the initial iodophor composition with a cascading waterfall stream produced by a cascading water sterilizer for the duration of the sterilization time period, optionally wherein the sterilization temperature is in a range from 65°C to 121°C, optionally wherein the sterilization time period is in a range from 1.5 minutes to 15 minutes.

15. The method of claim 14, further comprising terminating exposure to the sterilization temperature upon expiration of the sterilization time period.
